# FASCICULE DE BREVET EUROPEEN

(11) **EP 2 908 753 B1**
(45) Date de publication et mention de la délivrance du brevet: **07.12.2016**
(21) Numéro de dépôt: 13812010.0
(22) Date de dépôt: 17.10.2013
(51) Int. Cl.: A61B 90/00

(54) **SYSTEME DE FIXATION D'UN MATERIEL D'OSTEOSYNTHESE**
SYSTEM ZUM BEFESTIGEN EINES STÜCKS EINER OSTEOSYNTHESE-ANORDNUNG
SYSTEM FOR ATTACHING A PIECE OF OSTEOSYNTHESIS EQUIPMENT

(30) Priorité: 19.10.2012 FR 1259982
(43) Date de publication de la demande: 26.08.2015
(73) Titulaire: Teknimed, 65502 Vic-en-Bigorre Cedex (FR)
(72) Inventeur: LEONARD, Alain, 207 Nosy Be (MG); LEONARD, Carole, F-31380 Paulhac (FR); SENDER, Cyril, F-31500 Toulouse (FR); LIGNON, Olivier, F-81500 Ambres (FR); HALBIN, Gautier, F-31470 Fontenilles (FR); SAHRAOUI, Nouredine, F-31100 Toulouse (FR)
(74) Mandataire: Argyma
(86) Numéro de dépôt international: PCT/FR2013/052478
(87) Numéro de publication internationale: WO 2014/060702

(56) Documents cités:
- FR-A1- 2 932 078
- US-A- 2 940 488
- US-A- 5 524 512
- US-A1- 2006 079 903
- US-A1- 2007 005 077
- US-A1- 2010 274 298

## Description

La présente invention appartient au domaine des matériels chirurgicaux et se rapporte plus particulièrement à des systèmes de fixation utilisés dans la réparation osseuse. Elle a pour objet un système de fixation d'un matériel d'ostéosynthèse contre un tissu osseux, ce système comprenant au moins une vis de fixation et un ancillaire associé comprenant un tournevis.

L'ostéosynthèse regroupe l'ensemble des procédés qui permettent de maintenir en place deux structures osseuses suite à une fracture, une arthrodèse ou une ostéotomie. On y a recours par exemple quand la réduction (c'est-à-dire la remise des extrémités osseuses face à face) ne peut pas se faire par des manoeuvres extérieures ou lorsque les deux fragments ne sont pas stables. Elle met en oeuvre divers matériels permettant de maintenir entre eux les deux fragments de l'os (ou un os et un implant) comme des plaques, tiges, clous, vis, broches, agrafes, etc. Le but est d'arriver à la consolidation de l'os en position anatomique.

L'ostéosynthèse à l'aide d'un matériel tel qu'une plaque vissée par l'intermédiaire d'une ou de plusieurs vis est utilisée en chirurgie osseuse depuis des dizaines d'années. Le maintien de l'os est alors assuré par la plaque qui est fixée aux fragments osseux à réunir, à l'aide de plusieurs vis traversant ledit matériel et le tissu osseux. Les vis utilisées pour maintenir le matériel peuvent être de différents diamètres et de différentes longueurs en fonction de la forme du matériel et de l'emplacement d'implantation.

Ces vis comportent communément un relief à la partie supérieure de la tête, qui va coopérer avec un tournevis dont la pointe est de forme complémentaire : vis à tête fendue ou cruciforme, ou encore creuse (par exemple coopérant avec un tournevis 6 pans mâle). Elles sont mises à la disposition du chirurgien au moment de l'intervention en même temps qu'un ancillaire spécifique dédié à la mise en place du système de fixation, comprenant généralement des forets, un taraud et un tournevis.

L'ostéosynthèse par un matériel tel qu'une plaque associé à une ou des vis est considérée comme un moyen sûr pour obtenir une bonne consolidation osseuse, quand on peut la réaliser parfaitement. Pour cela, un certain nombre de conditions doivent être respectées, ou du moins recherchées, dans lesquelles la qualité du vissage joue un rôle essentiel. Il est indispensable que le matériel soit étroitement solidaire de l'os dès son implantation et dans la durée. Il est donc impératif que les vis restent en place malgré les vibrations ou autres sollicitations pouvant créer un jeu et conduire à leur descellement. Les vis doivent donc être solidement amarrées aussi bien à l'os qu'au matériel.

Il est également nécessaire de pouvoir orienter la vis selon un angle adapté que le chirurgien va choisir en fonction des paramètres osseux du patient. Les vis peuvent ainsi être insérées selon un axe perpendiculaire au matériel, mais il est souvent nécessaire de leur donner une certaine inclinaison par rapport à l'angle droit, notamment pour atteindre un fragment osseux éloigné de la zone de fracture. De ce fait, la tête de la vis ne peut pas s'emboîter totalement dans l'alvéole (la réservation) prévue à cet effet dans le matériel et fait saillie en surface.

Les dispositifs formés d'un matériel d'ostéosynthèse tel qu'une plaque et de vis proposés aux chirurgiens ont été pendant longtemps en métal, généralement en titane ou en acier inoxydable. A l'heure actuelle, les meilleurs dispositifs métalliques sont les dispositifs verrouillés et orientables. Chaque alvéole destinée à recevoir une tête de vis est alors constituée par un oeillet ayant une certaine mobilité par rapport au matériel. Le verrouillage est réalisé par un second filet ménagé à la partie inférieure de la tête de la vis, le premier filet correspondant au fût de la vis en prise avec l'os. Ce second filet vient se prendre dans un filet complémentaire ménagé au niveau de l'oeillet. Lorsque l'oeillet est monté orientable, ce dispositif de verrouillage peut en outre être incliné dans une direction appropriée, communément dans un rayon de l'ordre de 0 à 15° dans toutes les directions.

Les métaux ou alliages métalliques utilisés, bien que parfaitement tolérés par l'organisme, présentent deux inconvénients. D'une part, le matériel, fréquemment sous forme d'une plaque, n'étant que rarement retiré (environ dans 15% des cas), il va être impliqué dans la durée dans le fonctionnement cinématique de l'organe qu'il a permis de ressouder. Or, sa présence entraîne que les contraintes mécaniques ne sont pas transmises de façon homogène dans l'os concerné. Il en résulte une fragilisation de l'os au niveau des points de contact entre l'os et le matériel, à savoir essentiellement au niveau des vis. Les fractures secondaires sont donc fréquentes.

Un exemple de système de fixation d'un matériel d'ostéosynthèse contre un tissu osseux comportant une vis et un tournevis est décrit dans le document US 2006/0079903. Le tournevis de ce document comporte un manche, une tige dont l'extrémité avant porte un embout apte à coopérer avec la vis, et un manchon monté coulissant sur la tige, s'étendant autour d'une partie de la vis pour la guider en translation, et pouvant être bloqué le long de la tige.

Pour remédier à ce problème, des dispositifs d'ostéosynthèse comprenant un matériel par exemple sous forme d'une plaque et une ou des vis en polymères ont été proposés. Des polymères biocompatibles tels que le PEEK (polyétheréthercétone), les polyamides implantables, les UHMWPE (polyéthylène à masse molaire très élevée) ou encore les PET (polytéréphtalate d'éthylène), peuvent être utilisés. Ils offrent l'avantage d'une moindre rigidité, ce qui réduit les contraintes mécaniques imposées à l'os. Plus récemment, il a été proposé une alternative qui est fondée sur l'emploi de polymères résorbables qui présentent l'avantage de disparaître progressivement de l'organisme, au bout d'un laps de temps suffisant toutefois pour que l'os ait retrouvé sa solidité. On connaît par exemple les polymères appartenant à la famille des PLA (pour polylacticacids, ou en français acides polylactiques), des PCL (polycaprolactones), des PDS (polydioxanones) et des PGA (pour polyglycolacids, ou acides polyglycoliques en français), ainsi que des copolymères de ceux-ci.

Qu'ils soient résorbables ou non, tous ces polymères présentent un inconvénient majeur : ils sont radio-transparents. De ce fait, le contrôle de leur position durant leur implantation ou ultérieurement n'est pas possible avec les techniques radiographiques. Une nouvelle génération de dispositifs d'ostéosynthèse a récemment été élaborée par la demanderesse, qui sont réalisés à l'aide d'un mélange composite de matériaux à dégradation progressive comprenant un composé polymère ou copolymère et une charge minérale.apportée par une céramique.

On dispose donc désormais d'une gamme d'implants d'ostéosynthèse non métalliques offrant des avantages nouveaux au chirurgien. Cependant, un certain nombre d'inconvénients restent à surmonter.

On a vu que dans les dispositifs d'ostéosynthèse métalliques, la solidarité entre le matériel et la ou chaque vis est renforcée par un verrouillage. Or, les dispositifs réalisés à base de polymères (y compris d'un mélange composite en polymère et céramique) ne peuvent pas utiliser ce type de fixation. En effet, les techniques de formage des matériaux polymères ne permettent pas de réaliser un filet fin en surface, qui au demeurant ne résisterait pas aux contraintes mécaniques (essentiellement des frottements) lors du vissage. Les vis polymères connues à ce jour comportent une tête conique ou hémisphérique et sont placées perpendiculairement au plan général du matériel, fréquemment une plaque, ou avec un angle maximal de 5° par rapport à ce plan. Leur verrouillage comme pour les vis métalliques n'est pas possible. Ceci constitue un inconvénient notable des polymères face au métal.

Pour répondre à ce problème, il a été imaginé un système de maintien des matériels d'ostéosynthèse à base de polymères par des vis sans tête, également à base de polymères, celles-ci étant mises en place à l'aide d'un tournevis conçu et dédié spécifiquement pour ces vis.

Il est apparu qu'il était possible de réaliser un verrouillage des vis sur des matériels en polymères de synthèse en soudant les surfaces en contact. En effet, on peut mettre à profit la nature des matériaux utilisés pour, d'une part, sectionner la vis après insertion à une profondeur souhaitée dans l'os pour éliminer la partie arrière dépassant après insertion, et d'autre part, souder la vis et le matériel concomitant ensemble par une fusion superficielle des matériaux provoquée à l'aide d'un bistouri électrique. Le fût de la vis est ainsi fondu en surface dans l'oeillet du matériel qu'elle traverse.

Ce faisant, on se débarrasse de la partie arrière de la vis en faisant que la vis ne dépasse pas de la surface de l'os. La vis peut alors être insérée dans le matériel dans n'importe quelle direction avec une inclinaison élevée (pouvant aller jusqu'à 30°), sans créer une aspérité gênante, alors qu'un dispositif d'ostéosynthèse à vis métalliques admet des inclinaisons maximales de 15° seulement. On obtient ainsi, de manière inattendue, un dispositif verrouillé et orientable plus performant que le dispositif vis-matériel métallique.

Toutefois, un tel dispositif soulève un nouveau problème, qui est à la base de la présente invention. Il est en effet indispensable de contrôler la profondeur d'insertion des vis dans le tissu osseux. Ceci est particulièrement important du fait que, dans de nombreuses indications, il est recommandé que les vis traversent l'os de part en part pour agripper les parois externes de l'os cortical en deux points opposés. L'os cortical désigne la paroi externe des os qui leur confère rigidité et élasticité. Il est formé d'une couche dense de tissu calcifié qui entoure la cavité médullaire remplie de moelle osseuse. Une difficulté réside alors pour le chirurgien dans l'appréciation fine de la longueur d'insertion suffisante pour la fixation bicorticale, sans toutefois que la vis, dite vis bicorticale, ne dépasse excessivement de la surface de l'os.

De manière usuelle, lors d'une opération, le chirurgien va déterminer la longueur de vis nécessaire, notamment par l'intermédiaire d'une jauge de profondeur, puis choisir des vis de la longueur adéquate dans une gamme de vis à sa disposition et les placer en les vissant à fond jusqu'à ce que l'extrémité de vissage de la vis, le plus souvent sa tête, soit bloquée au contact de la plaque. Un tel procédé de fixation des vis implique l'utilisation de vis munies d'une tête, alors que dans le cas d'une vis destinée à être sectionnée, une telle procédure ne peut être suivie.

Le problème à la base de la présente invention est donc, pour un système de fixation d'un matériel d'ostéosynthèse contre un tissu osseux, ce système étant composé d'au moins une vis et d'un tournevis, de contrôler de manière précise la profondeur d'insertion de la vis dans le tissu osseux et dans le matériel d'ostéosynthèse appliqué contre ledit tissu.

A cet effet, la présente invention prévoit un système de fixation d'un matériel d'ostéosynthèse contre un tissu osseux, ce système comprenant au moins i) une vis comportant un fût fileté et une extrémité arrière, et ii) un tournevis comportant un manche prolongé par une tige dont l'extrémité avant porte un embout apte à coopérer avec l'extrémité arrière de la vis, système dans lequel la vis est constituée d'un matériau comprenant un polymère, et ledit tournevis est équipé d'un organe de guidage de la vis relié à la tige, une portion de l'organe de guidage faisant saillie en avant de l'embout du tournevis de sorte qu'elle s'étend le long ou autour d'une partie de la vis pour la guider en translation axiale, ledit organe de guidage présentant une extrémité avant réalisant une butée du tournevis contre la surface du matériel d'ostéosynthèse lorsque une profondeur prédéfinie de vissage de la vis dans le tissu osseux est atteinte.

Dans la description qui va suivre, et sauf indication différente, le système de fixation objet de l'invention sera présenté dans la configuration où la vis est en place sur le tournevis, et dans une orientation où sa partie avant pointe vers le matériel à fixer, tandis que sa partie arrière est celle qui est tournée vers l'opérateur. Les différents organes le composant seront également orientés selon cette convention.

Le tournevis permet de procéder au vissage d'une vis jusqu'à une profondeur choisie et prédéfinie, généralement relevée à l'aide d'une jauge de profondeur. La profondeur de vissage correspondra dans ce qui va suivre à la profondeur du trou pratiqué dans l'os, additionnée de l'épaisseur du matériel d'ostéosynthèse à travers lequel passe la vis. Cette profondeur de vissage est choisie et prédéfinie, généralement par le chirurgien : elle correspond au degré de pénétration de la vis pour assurer une fixation robuste.

Il est donc bien entendu que la profondeur de vissage de la vis se rapporte à la longueur de vis ayant progressé dans l'os (augmentée du l'épaisseur du matériel), ce qui a pour corollaire que l'on contrôle la progression de l'extrémité avant de la vis jusqu'à un niveau souhaité pour la fixation et qui peut être différent d'une fois à l'autre. Dans le système selon l'invention, ce n'est pas la longueur de la vis qui est choisie en fonction du degré d'insertion à atteindre. Et ce n'est pas non plus la position de l'extrémité arrière de la vis (arrivant à fleur du matériel d'ostéosynthèse à fixer) qui commande l'arrêt de l'avancement de la vis, comme c'est le cas dans d'autres systèmes connus, tels que celui décrit par exemple dans FR 2 932 078. Au contraire, selon l'invention la longueur de la vis est universelle, elle est enfoncée autant que souhaité, et c'est après la pose de la vis qu'on se débarrasse de la partie arrière excédentaire de la vis, en la sectionnant.

La vis est aisément sectionnée dans la mesure où elle est constituée d'un matériau comprenant un polymère, seul ou en mélange avec un autre matériau, dont la dureté est largement inférieure à celle des vis classiques en métal ou alliages métalliques.

Le système selon l'invention peut être utilisé à d'autres types d'assemblages que la fixation d'un matériel d'ostéosynthèse contre un tissu osseux. La vis est insérée dans l'organe de guidage jusqu'à ce que son extrémité arrière vienne en contact avec l'embout du tournevis. L'embout sera alors apte à coopérer avec l'extrémité arrière de la vis pour lui imprimer un mouvement de rotation lorsque l'utilisateur manipulera le tournevis.

Une portion de l'organe de guidage fait saillie en avant de l'embout du tournevis, de sorte que ladite portion s'étend en avant de l'embout, le long ou autour d'une partie de la vis, pour la guider en translation axiale, durant la phase de vissage. Un guidage en translation axiale est réalisé par une liaison linéaire annulaire, qui s'oppose aux translations transversales (radiales par rapport à l'axe de la tige). Tous les autres mouvements sont libres. Ce guidage est judicieusement assuré par l'extrémité avant de l'organe de guidage, à l'aide d'une liaison linéaire annulaire présentant un jeu suffisant pour laisser le mouvement de translation se faire librement, sans résistance. Le praticien peut exercer un effort sur le système sans craindre une déviation de l'outil ou une flexion indésirable de la vis.

L'organe de guidage remplit une seconde fonction, puisque son extrémité avant va réaliser une butée du tournevis contre la surface du matériel d'ostéosynthèse lorsque la profondeur de vissage souhaitée sera atteinte. En effet, conformément à l'usage, la vis et le tournevis coopèrent durant le vissage, mais se séparent une fois que la vis est placée. C'est communément l'opérateur qui apprécie de visu que la vis a entièrement pénétré dans la pièce à fixer, et qui, allégeant la pression qu'il exerce sur le manche du tournevis va en séparer la pointe de la tête de la vis. De manière originale selon l'invention, cette séparation se produit lorsque l'extrémité avant de l'organe de guidage étant arrivée au contact du matériel d'ostéosynthèse, le tournevis ne peut plus avancer. L'opérateur continuant à donner son mouvement de rotation au tournevis, la vis va quant à elle poursuivre son avancée, de sorte que son extrémité arrière va progressivement se dégager de l'embout du tournevis. La vis est totalement libérée et son avancée est stoppée quand au moment où sa pointe a atteint la profondeur initialement choisie. Même si l'opérateur persiste, la vis n'avancera plus.

Ainsi, conformément à la présente invention, l'organe de guidage qui forme butée contre la paroi du matériel d'ostéosynthèse est un élément indépendant de la vis. Il remplit deux fonctions en assurant, d'une part, le guidage de la vis pendant le vissage et d'autre part, l'arrêt du vissage à une profondeur prédéfinie, par désolidarisation de la vis et du tournevis. On peut de la sorte placer une vis avec une profondeur d'insertion précise et choisie, la profondeur étant préalablement déterminée par un moyen à la disposition du praticien (par exemple à l'aide d'une jauge de profondeur).

La profondeur de vissage une fois déterminée, on peut selon l'invention modifier la position de la butée en fonction de ladite profondeur. Il est possible de prévoir plusieurs positions de l'organe de guidage sur la tige correspondant à différentes profondeurs d'insertion de la vis. Selon une caractéristique préférée de l'invention, l'organe de guidage est monté coulissant sur la tige, des moyens de blocage en au moins une position dudit organe dans la longueur de la tige étant prévus. Cette position correspond à la profondeur prédéfinie de vissage de la vis et conditionne la portion dudit organe qu'il faut faire dépasser en avant de l'embout. On réalise alors une butée réglable par coulissement de l'organe de guidage sur la tige pour obtenir une longueur d'extension choisie.

Avantageusement, ledit organe de guidage est sous la forme d'un tube, dont l'extrémité avant est munie d'un orifice autorisant une translation axiale de la vis. Le tube est monté sur la tige qu'il entoure partiellement et sur laquelle il peut coulisser. Son extrémité avant qui fait fonction de butée, est percée d'un orifice laissant passer la vis. Elle peut être resserrée par rapport au corps du tube de façon à ne laisser que le jeu nécessaire à l'avancée de la vis tout en la maintenant efficacement dans l'axe de vissage.

De préférence, les moyens de blocage sont sous la forme d'une molette présentant un axe traversant l'organe de guidage et se bloquant sur la tige. Une molette est un moyen de solidarisation amovible de l'organe de guidage sur la tige adapté au réglage de la longueur de l'organe de guidage dépassant de l'embout du tournevis, en pouvant être facilement manipulée par le praticien par simple rotation.

Bien qu'il puisse être utilisé avec n'importe quel type de vis, le tournevis selon l'invention est tout indiqué pour placer une vis sans tête. C'est pourquoi, avantageusement, la vis utilisée dans le système selon l'invention est une vis sans tête, l'extrémité arrière de la vis ne faisant pas saillie latéralement par rapport au fût de la vis (au-delà de l'arrête du filetage). La section d'une telle vis a donc un diamètre identique ou au moins similaire à celui du fût sur toute sa longueur.

Cette caractéristique est avantageuse à plusieurs niveaux. D'une part, l'orifice de l'organe de guidage pourra être d'un diamètre à peine plus grand que celui de la vis de manière à assurer efficacement son guidage. D'autre part, l'absence de saillie sur la vis permet son passage intégral à travers l'orifice de l'organe de guidage. On peut non seulement mettre la vis en position sur le tournevis à travers l'orifice de l'organe de guidage sans avoir à le démonter, mais surtout, on peut retirer le tournevis et son organe de guidage après avoir procédé au vissage. Enfin, ce qui est particulièrement intéressant, la vis peut être vissée avec une inclinaison importante par rapport à la normale, sans qu'on soit gêné pour loger une tête de vis dans l'oeillet du matériel d'ostéosynthèse à fixer, puisque l'encombrement au niveau de l'oeillet du à une tête saillante est supprimé. Il est possible de réduire la taille des trous prévus sur le matériel d'ostéosynthèse, ce qui augmente sa rigidité comparée à un matériel avec des trous chanfreinés pour vis avec tête.

Selon une caractéristique préférée de l'invention, la vis est en matériau polymère, résorbable ou non, ou en mélange composite d'au moins un polymère et d'une céramique. La vis utilisée dans le système de fixation peut être constituée à 100% d'un polymère thermoplastique tel que du PEEK, PEKK, des polyamides implantables, des UHMWPE, les PET, ou encore en PLA, celui étant bio-résorbable. On peut aussi utiliser une vis fabriquée en matériau composite associant un polymère résorbable et une céramique résorbable. La céramique peut être choisie par exemple parmi les sulfates de calcium, les phosphates de calcium, le carbonate de calcium, les silicates notamment les bioverres, et sera préférentiellement de l'hydroxyapatite calcostroncique ou du tricalcium phosphate. L'intérêt de cette composition réside d'une part dans sa vitesse de dégradation plus rapide que celle d'un matériel d'ostéosynthèse en polymère seul, et d'autre part dans le fait qu'elle est radio-opaque.

Ces matières plastiques et/ou composites présentent l'avantage d'être facilement sectionnées. Le chirurgien pourra couper la partie arrière de la vis dépassant du site d'implantation à l'aide d'une simple pince par exemple, et n'aura pas à sélectionner une vis de longueur précise avant d'opérer. Quand la longueur de vis souhaitée aura pénétré dans l'os, il sera possible de sectionner la partie arrière excédentaire de la vis à ras du matériel d'ostéosynthèse, puis de la faire fusionner avec le matériel au niveau de l'oeillet, pour renforcer son verrouillage avec le matériel d'ostéosynthèse. Ainsi, seront fournies au chirurgiens des vis avec les différents diamètres utiles, mais en une seule longueur. Ceci limite le nombre de références mises à disposition du chirurgien.

Une coopération particulièrement intéressante peut donc être obtenue entre les vis sans tête en polymère ou composite, et le tournevis du système de fixation. On note cependant que les matériaux polymères ou composites n'étant pas aussi résistants que les métaux usuellement employés, il est préférable que l'extrémité arrière de la vis et l'embout du tournevis soient bien adaptés aux contraintes qu'ils vont subir, notamment pendant le vissage. C'est pourquoi il est proposé que la vis comporte en prolongement du fût fileté, une partie arrière permettant une association solide, bien que provisoire, de la vis et de du tournevis. Ainsi, selon un mode de réalisation particulier de l'invention, la vis présente une extension axiale non filetée prolongeant le fût fileté et se terminant par l'extrémité arrière, ladite extrémité arrière et l'embout adoptant des formes complémentaires mâle et femelle emboîtables. Ceci permet de ne munir la vis d'un filetage que sur une portion pour laquelle ce filetage est nécessaire.
Ceci permet aussi de donner à la partie arrière de la vis une épaisseur moindre que l'embout du tournevis, de sorte que celui-ci peut enserrer l'arrière de la vis pour la maintenir correctement, même si le matériau polymère a tendance à se déformer. C'est pourquoi, de préférence, l'extrémité arrière de la vis constitue la forme mâle et l'embout la forme femelle.

De manière encore préférée, l'extension arrière de la vis adopte la forme d'une spatule, c'est-à-dire d'une lame plus large qu'épaisse. Dans ce cas, l'embout du tournevis est de préférence un mandrin en U apte à recevoir l'extrémité de l'extension arrière de la vis. L'utilisation d'un mandrin est particulièrement bien adaptée à une solidarisation amovible avec l'extension arrière de la vis, mais restant ferme lors de la rotation du tournevis pour concourir à maintenir la vis lorsque celle-ci subit une torsion. En fin de vissage, lorsque l'extrémité avant de l'organe de guidage arrivera en butée, la spatule se dégagera spontanément et aisément des pâtes en U du mandrin, jusqu'à s'en séparer totalement, libérant ainsi l'ensemble du tournevis. Il suffira ensuite de retirer l'organe de guidage de la portion arrière de la vis dépassant encore, à sectionner cette dernière à ras de la plaque et à éventuellement la fusionner avec la plaque au moyen du bistouri électrique.

De préférence, dans le cas où l'embout du tournevis est un mandrin en U, cet embout est doté d'un manchon amovible, pour renforcer la résistance du mandrin durant le vissage.

L'embout du tournevis peut adopter des formes différentes, à condition que l'extrémité arrière de la vis soit conformée complémentairement. Il peut par ailleurs être interchangeable, ce qui permet de conserver le même corps du tournevis pour le vissage de vis de différents types en ne changeant que l'embout de vissage. Cela permet aussi d'utiliser le même corps de tournevis en lui associant d'autres outils pour des travaux autres que le vissage, par exemple pour le forage et/ou le taraudage du matériel et du tissu osseux.

Il est commode de pouvoir reporter la profondeur d'insertion de la vis, prédéfinie comme expliqué plus haut, directement sur un élément du tournevis visible du praticien. En effet, la position exacte de l'organe de guidage par rapport à la tige correspondant à la profondeur d'insertion désirée de la vis, il est avantageux de posséder des repères précis pour faire coulisser l'organe de guidage sur la tige juste à la longueur voulue. C'est pourquoi, avantageusement, la tige du tournevis comprend des moyens pour visualiser le réglage de la position de l'organe de guidage par rapport à la tige.

Pour ce faire, on peut par exemple réaliser l'organe de guidage en matériau transparent, de façon à ce que la tige reste apparente, celle-ci portant des repères dans sa longueur. On peut aussi prévoir une fenêtre ménagée dans l'organe de guidage. Selon un mode de réalisation préféré, les moyens d'affichage comprennent une réglette graduée portée par la tige, et une lumière ménagée dans l'organe de guidage permettant de voir au moins une partie de ladite réglette. La réglette peut comporter des graduations dans la même unité que celle qui est utilisée sur la jauge de profondeur. Une fois l'extension adéquate du système réalisée, on fixe l'organe de guidage sur la tige à l'aide du moyen de blocage retenu, par exemple une molette.

On note qu'il convient de tenir compte du mode d'association de l'embout et de l'arrière de la vis, en particulier dans le cas où l'embout adopte la forme d'un mandrin, celui-ci ayant une certaine profondeur. En effet, l'organe de guidage étant en arrivé butée, la vis est encore associée au mandrin. Elle va avancer encore un peu, de sorte que son extrémité arrière va se dégager progressivement de l'emprise du mandrin, pour finalement s'en libérer. La profondeur du mandrin doit donc être prise en compte dans la graduation de la réglette, en décalant l'origine d'autant, pour que la vis atteigne la profondeur préalablement mesurée avant dissociation de l'embout et de la vis.

Le tournevis du système selon l'invention comporte un manche pour sa prise en main par l'opérateur. Le manche du tournevis peut comprendre un organe de préhension monté en libre rotation. Le praticien peut ainsi poursuivre la rotation de l'outil sans relâcher sa pression sur le manche.

Grâce au système de fixation associant des vis et un tournevis adapté, il est désormais possible de poser des vis, avantageusement en polymère, de manière commode, solide et fiable dans le temps. Ceci autorise l'emploi de matériels d'ostéosynthèse en matériaux polymères sans plus soulever les difficultés qui limitaient jusqu'à présent leur utilisation massive en chirurgie. Le système est donc plus performant qu'un ensemble vis-matériel d'ostéosynthèse métallique dont les orientations maximales sont limitées.

La présente invention sera mieux comprise, et des détails en relevant apparaîtront, grâce à la description qui va être faite d'une de ses variantes de réalisation, en relation avec les figures annexées, dans lesquelles :
- La figure 1 représente un même tournevis et une vis faisant partie du système de fixation selon l'invention. Elle est déclinée en figures 1a, 1b et 1c dans lesquelles l'organe de guidage est plus ou moins engagé sur la tige.
- La figure 2 représente une vue éclatée du système selon l'invention, les pièces disposées à la distance qu'elles occupent vis-à-vis du site d'insertion de la vis.
- La figure 3 est une vue en perspective d'une vis faisant partie du système de fixation selon la présente invention.

### DESCRIPTION DU SYSTEME DE FIXATION

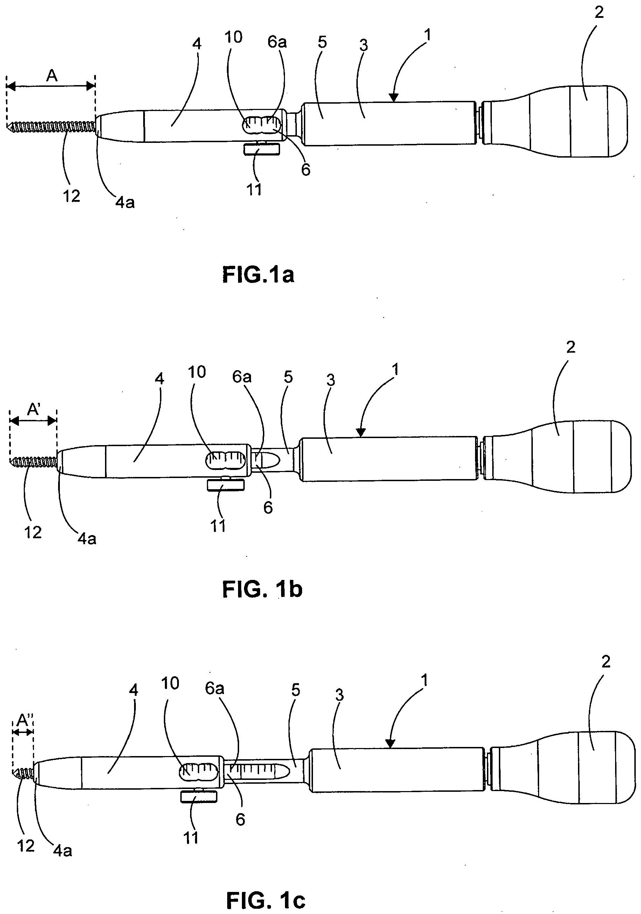

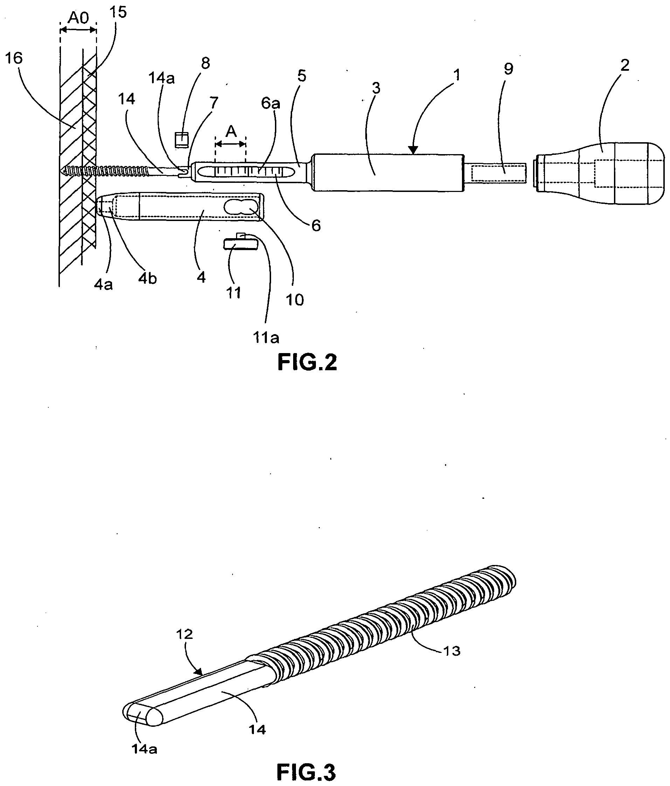

Sur les figures 1 et 2, il a été représenté un tournevis 1 et une vis 12 destinée à être vissée dans un tissu osseux 16 recevant un matériel d'ostéosynthèse, en l'occurrence une plaque 15. Le tournevis 1 et la vis 12 font partie du système de fixation d'un matériel d'ostéosynthèse selon la présente invention. La vis 12 présente un fût fileté 13 destiné à être inséré par vissage à travers la plaque d'ostéosynthèse 15 et le tissu osseux 16. Le fût fileté est prolongé par l'extension arrière 14 portant l'extrémité arrière 14a.

Le tournevis 1 comprend le manche 3 cylindrique s'étendant dans la longueur du tournevis 1. Le manche 3 est muni d'un élément de préhension 2, qui peut prendre différentes formes, par exemple en pommeau ou en T. Le manche 3 comporte un axe 9 le prolongeant à son extrémité tournée vers l'élément de préhension 2 et servant à l'emmanchement de ce dernier. Il est possible de motoriser le tournevis 1 en lui adjoignant des moyens de motorisation solidaires de l'axe 9.

Le manche 3 est prolongé par la tige 5 cylindrique l'extrémité avant de laquelle est monté l'embout 7 formant l'avant du tournevis 1. Dans ce qui va suivre, pour les éléments du tournevis 1, le terme avant qualifiera tout élément se trouvant en regard de la vis 12.

Durant son utilisation, la tige 5 du tournevis 1 est partiellement recouverte par l'organe de guidage 4 de la vis 12, qui sera précisément décrit plus loin. Cet organe de guidage 4 présente une portion qui dépasse vers l'avant au-delà de l'embout 7.

La tige 5 comporte sur une partie de sa surface externe des moyens d'affichage consistant en une lumière 10, laissant apparaître la réglette 6 portant des graduations, pour que le chirurgien, utilisateur du tournevis 1, règle la longueur de dépassement vers l'avant de la portion de l'organe de guidage 4 dépassant du tournevis 1, ceci afin d'obtenir une profondeur d'insertion voulue pour la vis 12.

L'extrémité avant du tournevis 1 porte l'embout 7 qui coopère avec l'extrémité arrière 14a de la vis 12. Dans certains modes de réalisation, l'embout 7 peut être amovible afin que le tournevis 1 puisse être muni de différentes formes de vissage coopérant avec différentes formes complémentaires prévues sur l'extrémité arrière 14a de la vis 12.

L'embout 7 présente un évidement central recevant l'extrémité arrière 14a, cet évidement permettant de solidariser temporairement la vis 12 avec le tournevis 1 lors du vissage. A la figure 2, l'embout 7 est sous la forme d'un mandrin en U recevant l'extrémité arrière 14a et permettant son maintien quand la vis 12 subit une torsion. L'embout 7 est recouvert du manchon 8 de protection l'entourant tout en permettant le passage de l'extrémité arrière de la vis 12 dans l'embout 7. Le manchon 8 peut être amovible ou au contraire monté fixe, par exemple par soudage.

L'organe de guidage 4 adopte la forme d'un tube creux qui recouvre partiellement en l'entourant une partie de la tige 5 du tournevis 1, et dépasse à l'avant du tournevis 1, de sorte qu'il peut recevoir en son intérieur une partie de la vis 12. L'organe de guidage 4 à son extrémité avant 4a est muni d'un orifice 4b suffisant pour autoriser l'avancée de la vis 12. Il sert ainsi au guidage de la vis 12 en translation axiale, la vis 12 y pénétrant par son ouverture avant 4a quand le tournevis 1 est mis en position de vissage. L'organe de guidage 4 sert aussi de butée pour le tournevis 1 avec son extrémité avant 4a butant contre la paroi de la plaque d'ostéosynthèse 15 quand une profondeur prédéfinie d'insertion de la vis 12 dans le tissu osseux 16 a été atteinte. Ladite profondeur d'insertion ne peut être dépassée puisque la vis 12 et le tournevis 1 sont désaccouplés du fait de la mise en butée de l'organe de guidage 4 contre la plaque 15.

Comme montré aux figures 1 à 3, l'organe de guidage 4 est avantageusement sous la forme d'un tube recouvrant une partie de la vis 12 et de la tige 5. Il peut coulisser le long de la tige 5 du tournevis 1. Il comporte la molette 11 présentant l'axe 11 a qui se fixe radialement par vissage contre la tige 5 du tournevis 1, en traversant la paroi de l'organe de guidage 4. L'organe de guidage peut ainsi être bloqué dans une position choisie. La lumière 10 ménagée sur l'organe de guidage 4 permet au praticien de voir les graduations 6a de la réglette 6 lors du positionnement puis du blocage de l'organe de guidage 4 sur la tige 5.

Le système de fixation selon l'invention peut comprendre plusieurs types de vis, de différentes tailles et/ou de différentes formes. La vis présentée dans le présent exemple de réalisation (figure 3) de l'invention est une vis sans tête. La vis 12 comprend le fût fileté 13 prolongé par une extension axiale 14 non fileté, portant l'extrémité arrière 14a ne faisant pas saillie latéralement par rapport au fût de la vis 12. Le fût fileté 13 présente une section circulaire, l'extension arrière 14 présentant quant à elle une section sensiblement rectangulaire, ce qui lui confère la forme d'une spatule. Cette forme est tout indiquée pour coopérer avec l'embout 7 ayant la forme d'un mandrin en U.

Il est aussi possible de prévoir une extension arrière 14 circulaire ou un fût fileté 13 circulaire sans extension axiale. Dans ces deux cas, l'extrémité arrière14a de la vis 12 peut présenter au moins une partie en saillie axiale ou au moins un logement de réception coopérant respectivement avec un logement de réception ou une partie en saillie axiale prévu sur l'embout du tournevis.

### UTILISATION DU SYSTEME DE FIXATION

Les figures 1a, 1b et 1c montrent le même tournevis mais dont le réglage est différent. Une vis identique dans les trois cas a été installée sur le tournevis, en l'enfonçant à travers l'orifice 4b dans l'organe de guidage 4, jusqu'à ce que son extrémité arrière 14a soit parvenue dans l'embout 7. Comme on le voit, la longueur de la vis (notée A, A' et A") dépassant de l'extrémité avant 4a de l'organe de guidage 4 est en relation avec le degré de rétractation de l'organe de guidage 4 sur la tige 5.

La longueur de dépassement de l'organe de guidage 4 à l'avant du tournevis 1 peut ainsi être précisément fixée. En pratique, on relève la profondeur du trou devant recevoir la vis 12, à l'aide d'un outil tel qu'une jauge de profondeur, et selon cette profondeur d'insertion ainsi prédéfinie de la vis, on fait coulisser et on bloque l'organe de guidage 4 à la position correspondante sur la tige 5, repérée par lecture directe sur la réglette. La profondeur d'insertion souhaitée de la vis 12 dans le plaque 15 et le tissu 16 est ainsi soustraite de la longueur de la vis 12 par le réglage du positionnement de l'organe de guidage 4.

Il faut en outre tenir compte du mode d'association de l'embout et de l'arrière de la vis, en particulier dans le cas où l'embout adopte la forme d'un mandrin, celui-ci ayant une certaine profondeur. L'organe de guidage étant en arrivé butée, la vis va avancer encore un peu de sorte que l'extrémité arrière de la vis va se dégager progressivement de l'emprise du mandrin, pour finalement s'en libérer totalement. La profondeur du mandrin doit être prise en compte dans la graduation de la réglette, en décalant l'origine d'autant.

A la figure 2, la représentation éclatée du système selon l'exemple fait apparaître la correspondance entre la profondeur Ao de pénétration de la vis travers la plaque 15 et dans l'os 16, et la position du curseur 6a sur la réglette 6 à la distance A de rétractation de l'organe de guidage. Lors du vissage dans le tissu osseux 16, la vis 12 s'enfonce à travers le plaque 15 puis le tissu osseux 16, de sorte que l'extrémité avant 4a de l'organe de guidage 4 se rapproche de la paroi de la plaque 15. Lorsque la vis 12 a pénétré à la profondeur Ao (diminuée de la longueur de pénétration de l'arrière de la vis dans le mandrin), l'extrémité avant 4a de l'organe de guidage 4 arrive en butée contre la plaque 15. Une rotation supplémentaire du tournevis 1 va produire une avancée de la vis, alors que le tournevis est maintenant bloqué, provoquant le retrait progressif puis la séparation de l'extrémité arrière 14a de la vis et de l'embout 7. Un calibrage fin permet de tenir compte du fait que le matériau de la vis va se déformer sous la force de torsion subie, et de la profondeur des pattes du mandrin. La réglette 10 est donc étalonnée pour correspondre exactement à la profondeur Ao prédéterminée.

Pour obtenir une fixation bicorticale efficace, comme illustré à la figure 2, la vis 12 traverse entièrement l'épaisseur du plaque 15 et du tissu 16, mais elle ne doit pas faire saillie trop loin de l'autre côté du tissu osseux. Ceci est désormais possible grâce au système selon l'invention. Ce qui a été décrit précédemment est valable pour toutes les profondeurs d'insertion par vissage de la vis 12 dans une plaque d'ostéosynthèse 15 ou autre matériel, et un tissu osseux 16.

Les vis 12 peuvent être de compositions variées, par exemple composées jusqu'à 100% d'au moins un polymère. On peut utiliser des acides polylactiques (ou PLA) afin qu'elles présentent une élasticité leur permettant de se coincer dans le plaque d'ostéosynthèse 15. De manière générale, les vis 12 peuvent être en polymère résorbable ou non, ou en un mélange de polymères résorbables ou non, ou en un mélange composite d'au moins un polymère et d'une ou de plusieurs céramiques.

Le système est particulièrement adapté à la fixation de plaque, ou autre matériel, en polymère, résorbable ou non, à l'aide de vis également composées de tels matériaux, comme il a été exposé plus haut. Une fois la vis insérée, et le tournevis retiré, on sectionne la partie restante à ras de la plaque, ce qui est possible aisément dès lors que le matériaux composant la vis est de type polymère ou composite à base de polymère. La force d'arrachement de la vis 12 prise en force dans la plaque 15 et le tissu osseux 16 est alors identique à la force d'arrachement d'une tête de vis. Pour obtenir un verrouillage de la vis 12 dans le plaque 15 encore meilleur, il est possible de souder la vis 12 et la plaque 15 à chaud, par exemple à l'aide d'un bistouri électrique.

## Revendications

1. Système de fixation d'un matériel d'ostéosynthèse contre un tissu osseux (16), ce système comprenant au moins :
- i) une vis (12) comportant un fût fileté (13) et une extrémité arrière (14a), et
- ii) un tournevis (1) comportant un manche (3) prolongé par une tige (5) dont l'extrémité avant porte un embout (7) apte à coopérer avec l'extrémité arrière (14a) de la vis (12), ledit tournevis étant équipé d'un organe de guidage (4) de la vis (12) relié à la tige (5) et présentant une extrémité avant (4a), une portion de l'organe de guidage (4) faisant saillie en avant de l'embout (7) du tournevis (1) et s'étendant le long ou autour d'une partie de la vis (12) pour la guider en translation axiale, et l'organe de guidage (4) étant monté coulissant sur la tige (5), des moyens de blocage en au moins une position dudit organe (4) dans la longueur de la tige (5) étant prévus,
**caractérisé en ce que** la vis (12) est constituée d'un matériau comprenant un polymère, la vis est insérée libre en translation dans l'organe de guidage, et l'organe de guidage (4) est bloqué par lesdits moyens de blocage en une position dans la longueur de la tige (5) correspondant à une profondeur prédéfinie de vissage de la vis (12), ladite extrémité avant (4a) de l'organe de guidage (4) réalisant une butée du tournevis (1) contre la surface dudit matériel d'ostéosynthèse lorsque une profondeur prédéfinie de vissage de la vis (12) dans le tissu osseux (16) est atteinte, provoquant le blocage de l'avancée du tournevis (1) puis permettant l'arrêt du vissage par séparation de l'extrémité arrière (14a) de la vis (12) et de l'embout (7).

2. Système selon la revendication 1, **caractérisé en ce que** ledit organe de guidage (4) est sous la forme d'un tube dont l'extrémité avant (4a) est munie d'un orifice (4b) autorisant une translation axiale de la vis (12).

3. Système selon la revendication 1 ou 2, **caractérisé en ce que** les moyens de blocage de l'organe de guidage (4) sont sous la forme d'une molette (11) présentant un axe (11a) traversant l'organe de guidage (4) et se bloquant sur la tige (5).

4. Système selon l'une quelconque des revendications 1 à 3, **caractérisé en ce que** la vis (12) est une vis sans tête, l'extrémité arrière (14a) de la vis (12) ne présentant pas de saillie latérale.

5. Système selon l'une quelconque des revendications précédentes, **caractérisé en ce que** la vis (12) est en matériau polymère, résorbable ou non, ou en mélange composite polymère et céramique.

6. Système selon la revendication précédente, **caractérisé en ce que** la vis (12) présente une extension axiale (14) non filetée prolongeant le fût fileté (13) et se terminant par l'extrémité arrière (14a), ladite extrémité arrière (14a) et l'embout (7) du tournevis (1) adoptant des formes complémentaires mâle et femelle emboîtables.

7. Système selon la revendication précédente, **caractérisé en ce que** l'extrémité arrière (14a) de la vis (12) constitue la forme mâle et l'embout (7) la forme femelle.

8. Système selon la revendication précédente, **caractérisé en ce que** l'extension arrière (14) de la vis (12) adopte la forme d'une spatule.

9. Système selon l'une quelconque des revendications précédentes, **caractérisé en ce que** l'embout (7) est un mandrin en U apte à recevoir l'extrémité (14a) de l'extension arrière (14) de la vis (12).

10. Système selon la revendication précédente, **caractérisé en ce que** l'embout (7) est doté d'un manchon de renfort (8) amovible.

11. Système selon l'une quelconque des revendications 1 à 10, **caractérisé en ce que** la tige (5) du tournevis (1) comprend des moyens pour visualiser le réglage de la position de l'organe de guidage (4) par rapport à la tige (5).

12. Système selon la revendication 11, caractérisé en ce qu'il comprend une réglette graduée (6) portée par la tige (5), et une lumière (10) ménagée dans l'organe de guidage (4) permettant de voir au moins une partie de ladite réglette (6).

13. Système selon l'une quelconque des revendications précédentes, **caractérisé en ce que** le manche (3) du tournevis (1) comprend un organe de préhension (2) monté en libre rotation.

## Patentansprüche

1. System zur Fixierung eines Osteosynthesebauteils an einem Knochengewebe (16), wobei das System mindestens Folgendes umfasst:
- i) eine Schraube (12), die einen Gewindeschaft (13) und ein hinteres Ende (14a) aufweist, und
- ii) einen Schraubendreher (1), der einen Griff (3) aufweist, welcher sich in einem Stift (5) fortsetzt, dessen Ende mit einem Ansatzstück (7) versehen ist, das dazu befähigt ist, mit dem hinteren Ende (14a) der Schraube (12) zusammenzuwirken, wobei der Schraubendreher mit einem Organ zur Führung (4) der Schraube (12) ausgestattet ist, welches mit dem Stift (5) verbunden ist und ein vorderes Ende (4a) aufweist, wobei ein Abschnitt des Führungsorgans (4) vorderseitig über das Ansatzstück (7) des Schraubendrehers (1) hinausragt und sich um einen Teil der Schraube (12) herum oder entlang dieser erstreckt, um sie gemäß einer axialen Linearbewegung zu führen, und wobei das Führungsorgan (4) gleitend an dem Stift (5) gelagert ist, wobei Mittel vorgesehen sind, die das Organ (4) an mindestens einer Position entlang des Stiftes (5) blockieren können,
**dadurch gekennzeichnet, dass** die Schraube (12) aus einem Material besteht, welches ein Polymer umfasst, dass die Schraube derart in das Führungsorgan eingeführt wird, dass sie ungehindert eine Linearbewegung ausführen kann, und dass das Führungsorgan (4) von den Blockiermitteln an einer Position entlang des Stiftes (5) blockiert wird, welche einer vorbestimmten Einschraubtiefe der Schraube (12) entspricht, wobei der Schraubendreher (1) mit dem vorderen Ende (4a) des Führungsorgans (4) gegen die Oberfläche des Osteosynthesebauteils stößt, wenn die vorbestimmte Tiefe des Einschraubens der Schraube (12) in das Knochengewebe (16) erreicht ist, wodurch die Vorwärtsbewegung des Schraubendrehers (1) blockiert wird, woraufhin der Schraubvorgang dadurch abgebrochen werden kann, dass das hintere Ende (14a) der Schraube (12) von dem Ansatzstück (7) getrennt wird.

2. System nach Anspruch 1, **dadurch gekennzeichnet, dass** das Führungsorgan (4) in Form einer Röhre ausgeführt ist, deren vorderes Ende (4a) mit einer Öffnung (4b) versehen ist, die eine axiale Linearbewegung der Schraube (12) gestattet.

3. System nach Anspruch 1 oder 2, **dadurch gekennzeichnet, dass** die Mittel zum Blockieren des Führungsorgans (4) in Form eines drehbaren Elements (11) ausgeführt ist, das eine Achse (11 a) aufweist, welche das Führungsorgan (4) durchquert und blockierend mit dem Stift (5) zusammenwirkt.

4. System nach einem beliebigen der Ansprüche 1 bis 3, **dadurch gekennzeichnet, dass** es sich bei der Schraube (12) um eine kopflose Schraube handelt, wobei das hintere Ende (14a) der Schraube (12) keinerlei seitlichen Vorsprung aufweist.

5. System nach einem beliebigen der vorhergehenden Ansprüche, **dadurch gekennzeichnet, dass** die Schraube (12) aus einem Polymermaterial, welches resorbierbar ist oder nicht, oder aus einer Polymer/Keramik-Verbundmischung ist.

6. System nach dem vorhergehend Anspruch, **dadurch gekennzeichnet, dass** die Schraube (12) eine axiale Verlängerung (14) aufweist, die gewindelos ist, sich an den Gewindeschaft (13) anschließt und in dem hinteren Ende (14a) endet, wobei das hintere Ende (14a) und das Ansatzstück (7) des Schraubendrehers (1) komplementäre Formen haben, die nach dem Stecker-Buchse-Prinzip ineinandergesteckt werden können.

7. System nach dem vorhergehenden Anspruch, **dadurch gekennzeichnet, dass** das hintere Ende (14a) der Schraube (12) die Steckerform und das Ansatzstück (7) die Buchsenform darstellt.

8. System nach dem vorhergehenden Anspruch, **dadurch gekennzeichnet, dass** die rückseitige Verlängerung (14) der Schraube (12) die Form eines Spatels hat.

9. System nach einem beliebigen der vorhergehenden Ansprüche, **dadurch gekennzeichnet, dass** es sich bei dem Ansatzstück (7) um ein U-förmiges Haltefutter handelt, welches das Ende (14a) der rückseitigen Verlängerung (14) der Schraube (12) aufnehmen kann.

10. System nach dem vorhergehenden Anspruch, **dadurch gekennzeichnet, dass** das Ansatzstück (7) mit einer abnehmbaren Verstärkungsmanschette (8) versehen ist.

11. System nach einem beliebigen der Ansprüche 1 bis 10, **dadurch gekennzeichnet, dass** der Stift (5) des Schraubendrehers (1) Mittel umfasst, welche die Einstellung der Position des Führungsorgans (4) unter Bezugnahme auf den Stift (5) anzeigen.

12. System nach Anspruch 11, **dadurch gekennzeichnet, dass** es eine skalierte Leiste (6) umfasst, die sich auf dem Stift (5) befindet, sowie eine Öffnung (10), die in dem Führungsorgan (4) ausgespart ist, um den Blick auf mindestens einen Teil der Leiste (6) freizugeben.

13. System nach einem beliebigen der vorhergehenden Ansprüche, **dadurch gekennzeichnet, dass** der Griff (3) des Schraubendrehers (1) ein Halteorgan (2) umfasst, das frei drehbar gelagert ist.

## Claims

1. System for fixation of an osteosynthesis instrument against a bone tissue (16), this system comprising at least :
- i) a screw (12) having a threaded shaft (13) and a rear end (14a), and
- ii) a screwdriver (1) having a handle (3) extended by a rod (5), the end of which carries an adapter (7) suitable for cooperating with the rear end (14a) of the screw (12), said screwdriver being equipped with a guide member (4) of the screw (12) connected to the rod (5) and having a front end (4a), with one portion of the guide member (4) projecting in front of the adapter (7) of the screwdriver (1) and extending along or around a part of the screw (12) in order to guide it in axial translation, and said guide member (4) being mounted so as to slide on the rod (5), means being provided for locking said member (4) in at least one position in the length of the rod (5),
**characterized in that** the screw (12) consist of a material comprising a polymer, the screw is inserted freely in translation into the guiding member, and the guiding member (4) is locked by said locking means in one position in the length of the rod (5), corresponding to one predefined screwing depth of the screw (12), said front end (4a) of the guide member (4) forming a stop of the screwdriver (1) against the surface of said osteosynthesis instrument when a predefined screwing depth of the screw (12) in the bone tissue (16) is reached, causing the screwdriver (1) to be blocked and allowing the screw advance to stop by separation of the rear end (14a) of the screw (12) from the adapter (7).

2. System according to Claim 1, **characterized in that** said guide member (4) is in the form of a tube, the front end (4a) of which is provided with an orifice (4b) allowing axial translation of the screw (12).

3. System according to Claim 1 or 2, **characterized in that** the means for locking the guide member (4) are in the form of a knurled wheel (11) having an axle (11 a) which passes through the guide member (4) and is blocked on the rod (5).

4. System according to any of Claims 1 to 3, **characterized in that** the screw (12) is a headless screw (12), the rear end (14a) of the screw (12) not having a lateral projection.

5. System according to any of the preceding claims, **characterized in that** the screw (12) is made of optionally resorbable polymer material, or of a composite polymer and a ceramic mixture.

6. System according to the preceding claim, **characterized in that** the screw (12) has an unthreaded axial extension (14) continuing the threaded shaft (13) and ending at the rear end (14a), said rear end (14a) and the adapter (7) of the screwdriver (1) having complementary engageable male and female shapes.

7. System according to the preceding claim, **characterized in that** the rear end (14a) of the screw (12) constitutes the male shape and the adapter (7) constitutes the female shape.

8. System according to the preceding claim, **characterized in that** the rear extension (14) of the screw (12) has the shape of a spatula.

9. System according to any of the preceding claims, **characterized in that** the adapter (7) is a U-shaped mandrel suitable for receiving the end (14a) of the rear extension (14) of the screw (12).

10. System according to the preceding claim, **characterized in that** the adapter (7) is provided with a removable reinforcing sleeve (8).

11. System according to any of Claims 1 to 10, **characterized in that** the rod (5) of the screwdriver (1) comprises means for visualizing the adjustment of the position of the guide member (4) with respect to the rod (5).

12. System according to Claim 11, **characterized in that** it comprises a graduated scale (6) carried by the rod (5), and a slot (10) formed in the guide member (4) making it possible to see at least a part of said scale (6).

13. System according to any one of the preceding claims, **characterized in that** the handle (3) of the screwdriver (1) comprises a holding member (2) mounted freely in rotation.
